# EUROPEAN PATENT APPLICATION

(11) **EP 4 467 147 A1**
(43) Date of publication of application: **27.11.2024**
(21) Application number: 23743449.3
(22) Date of filing: 18.01.2023
(51) Int. Cl.: A61K 35/74, A61K 35/745, A61P 29/00, A23L 33/135, A23K 10/16, A61P 1/00

(54) **COMPOSITION FOR PREVENTING, AMELIORATING, OR TREATING INFLAMMATORY DISEASES COMPRISING EXTRACELLULAR VESICLES DERIVED FROM ROSEBURIA SPP. OR BIFIDOBACTERIUM SPP**

(30) Priority: 20.01.2022 KR 20220008554; 17.01.2023 KR 20230006807
(71) Applicant: Korea Institute of Science and Technology, Seoul 02792 (KR)
(72) Inventor: CHOI, Ki Young, Gangneung-si, Gangwon-do 25451 (KR); CHA, Kwang-Hyun, Gangneung-si, Gangwon-do 25451 (KR); HAN, Hwa Seung, Gangneung-si, Gangwon-do 25451 (KR); PARK, Jin Soo, Gangneung-si, Gangwon-do 25451 (KR); SONG, Dae-geun, Gangneung-si, Gangwon-do 25451 (KR); KIM, Myung Suk, Gangneung-si, Gangwon-do 25451 (KR); KWON, Hak Cheol, Gangneung-si, Gangwon-do 25451 (KR)
(74) Representative: Dehns
(86) International application number: PCT/KR2023/000838
(87) International publication number: WO 2023/140602

(57) **Abstract**

The present invention relates to a composition for preventing, ameliorating, or treating inflammatory diseases comprising extracellular vesicles (EVs) derived from *Roseburia* spp. strains and/or *Bifidobacterium* spp. strains. The EVs of the present invention exhibits effectiveness in improving inflammatory bowel disease (IBD), and additionally, all EVs derived from congeneric strains corresponding to *Roseburia* spp. strains or *Bifidobacterium* spp. strains. have been confirmed to exhibit strong anti-inflammatory effect. As such, the strain of the present invention and EVs derived therefrom can be provided as a composition for preventing, ameliorating, or treating a variety of inflammatory diseases.

## Description

### [Technical Field]

The present invention relates to a composition for preventing, ameliorating or treating inflammatory diseases, including a *Roseburia* spp. strain, a *Bifidobacterium* spp. strain, or extracellular vesicles (EVs) derived therefrom.

The present invention claims priority based on Korean Patent Application No. 10-2022-0008554 filed on January 20, 2022 and Korean Patent Application No. 10-2023-0006807 filed on January 17, 2023, and all contents disclosed in the specification and drawings of the above applications are incorporated in the present application by reference.

### [Background Art]

Inflammation refers to the pathological conditions of an abscess formed by the invasion of external infectious agents (bacteria, fungi, viruses, various types of allergens). Specifically, when external bacteria invade and proliferate in a specific tissue, the leukocytes of the body recognize this and actively attack the proliferated external bacteria. During this process, the dead cells of the leukocytes generated accumulate in the tissue invaded by the bacteria, and at the same time, the cell lysate of the invading bacteria killed by the leukocytes is dissolved into the invaded tissue to form an abscess. The treatment of abscesses due to inflammation may be promoted through an anti-inflammatory action, which is an inflammatory treatment promoting action, such as inhibiting the proliferation of invading bacteria using antibacterial agents or activating macrophages that phagocytize foreign substances accumulating in abscesses to enhance the functions of the macrophages to digest and excrete the foreign substances.

In general, an inflammatory response is a biological defense reaction process for repairing and regenerating damage caused by invasion that brings about organic changes in cells or tissues of the living body, and local blood vessels and various tissue cells and immune cells of body fluids act in this reaction process. The inflammatory response normally induced by external invading bacteria is a defense system to protect the living body, but when an abnormally excessive inflammatory response is induced, various diseases occur, and these diseases are referred to as inflammatory diseases. In inflammatory diseases, various inflammatory mediators secreted from target cells activated by external stimuli amplify and sustain inflammation, which can threaten the life of the human body. Inflammatory diseases include acute bronchitis, chronic bronchitis, acute bronchiolitis, chronic bronchiolitis, sepsis, septic shock, acute respiratory distress syndrome, multiple organ failure, and chronic obstructive pulmonary disease. Inflammatory bowel disease (IBD), one of the representative inflammatory diseases, is a disease that causes chronic inflammation in the gastrointestinal tract, shows a chronic course, gradually progresses, causes complications, and reduces physical functions (SY Na and W Moon, Korean J Gastroenterol Vol. 71 No. 2, 61-68). IBD is mainly classified into two forms: ulcerative colitis (UC) and Crohn's disease (CD). The exact pathogenesis of IBD is not known, and IBD is presumed to be caused by a combination of genetic factors and environmental factors such as stress and drug use. Therefore, since each patient has a different etiology and exhibits a different course and prognosis, UBD is a disease that is difficult to completely cure.

The onset of many inflammation-related diseases is associated with the activation of macrophages and the consequent excessive production of inflammation-related factors, and representative inflammation-related factors include IL-1β and TNF-α. In addition, among the inducers of the inflammatory response, lipopolysaccharide (LPS) is a substance constituting the cell surface of Gram-negative bacteria, and it interacts with immune cells such as leukocytes and is involved in the regulation of inflammatory-related cytokines. In particular, inflammatory cytokines such as IL-1βTNF-α and IL-6 play an important role in mediating the pathological progression of various infectious or inflammatory diseases. These inflammatory cytokines are produced by various immune and inflammatory cells and contribute to the recruitment of additional inflammatory cells to the site of injury resulting in tissue and organ damage.

### [Disclosure]

### [Technical Problem]

As a result of diligent efforts to find a drug having a better effect on inflammatory diseases than conventional therapeutic agents, the inventors of the present invention have completed the present invention by confirming that extracellular vesicles (EVs) derived from *Roseburia* spp. strains and *Bifidobacterium* spp. strains exhibit excellent prevention, amelioration, and treatment effects on inflammatory diseases.

Therefore, an object of the present invention is to provide a pharmaceutical composition for preventing or treating inflammatory diseases, including one or more selected from the group consisting of EVs derived from a *Roseburia* spp. strain and EVs derived from a *Bifidobacterium* spp. strain as an active ingredient.

Another object of the present invention is to provide a kit for preventing or treating inflammatory diseases, including one or more selected from the group consisting of EVs derived from a *Roseburia* spp. strain and EVs derived from a *Bifidobacterium* spp. strain as an active ingredient.

Still another object of the present invention is to provide a method of preventing or treating inflammatory diseases, including administering one or more selected from the group consisting of EVs derived from a *Roseburia* spp. strain and EVs derived from a *Bifidobacterium* spp. strain to a subject in need thereof.

Yet another object of the present invention is to provide a food composition for preventing or ameliorating inflammatory diseases, including one or more selected from the group consisting of EVs derived from a *Roseburia* spp. strain and EVs derived from a *Bifidobacterium* spp. strain.

Yet another object of the present invention is to provide a feed composition for preventing or ameliorating inflammatory diseases, including one or more selected from the group consisting of EVs derived from a *Roseburia* spp. strain and EVs derived from a *Bifidobacterium* spp. strain.

However, the technical problem to be solved by the present invention is not limited to the above-mentioned problems, and other problems not mentioned will be clearly understood by those skilled in the art from the description below.

### [Technical Solution]

The present invention provides a pharmaceutical composition for preventing or treating inflammatory diseases, including one or more selected from the group consisting of EVs derived from a *Roseburia* spp. strain and EVs derived from a *Bifidobacterium* spp. strain as an active ingredient.

In addition, the present invention provides a food composition for preventing or ameliorating inflammatory diseases, including one or more selected from the group consisting of EVs derived from a *Roseburia* spp. strain and EVs derived from a *Bifidobacterium* spp. strain as an active ingredient.

In addition, the present invention provides a feed composition for preventing or ameliorating inflammatory diseases, including one or more selected from the group consisting of EVs derived from a *Roseburia* spp. strain and EVs derived from a *Bifidobacterium* spp. strain as an active ingredient.

In addition, the present invention provides a kit for preventing, ameliorating, or treating inflammatory diseases, including one or more selected from the group consisting of EVs derived from a *Roseburia* spp. strain and EVs derived from a *Bifidobacterium* spp. strain.

Furthermore, the present invention provides a method of preventing or treating inflammatory diseases, including administering one or more selected from the group consisting of EVs derived from a *Roseburia* spp. strain and EVs derived from a *Bifidobacterium* spp. strain to a subject in need thereof.

Furthermore, the present invention provides a use of one or more selected from the group consisting of EVs derived from a *Roseburia* spp. strain and EVs derived from a *Bifidobacterium* spp. strain for preventing or treating inflammatory diseases.

Furthermore, the present invention provides a use of one or more selected from the group consisting of EVs derived from a *Roseburia* spp. strain and EVs derived from a *Bifidobacterium* spp. strain for preparing a therapeutic agent for inflammatory diseases.

In one embodiment of the present invention, the average diameter of the EVs may be 30 nm to 200 nm, but is not limited thereto.

In another embodiment of the present invention the EVs derived from a *Roseburia* spp. strain may be naturally or artificially secreted from a *Roseburia* spp. strain; and
in still another embodiment of the present invention, the EVs derived from a *Bifidobacterium* spp. strain may be naturally or artificially secreted from a *Bifidobacterium* spp. strain, but are not limited thereto.

In yet another embodiment of the present invention, the *Roseburia* spp. strain may be one or more selected from the group consisting of *Roseburia intestinalis, Roseburia faecis, Roseburia hominis,* and *Roseburia inulinivorans,* but is not limited thereto.

In yet another embodiment of the present invention, the *Bifidobacterium* spp. strain may be one or more selected from the group consisting of *Bifidobacterium longum, Bifidobacterium bifidum, Bifidobacterium breve, Bifidobacterium lactis,* and *Bifidobacterium adolescentis,* but is not limited thereto.

In yet another embodiment of the present invention, the inflammatory disease may be one or more selected from the group consisting of gastritis, gastric ulcers, duodenal ulcers, inflammatory skin disease, allergic diseases, irritable bowel syndrome, ulcerative colitis, inflammatory bowel disease, peritonitis, osteomyelitis, cellulitis, meningitis, encephalitis, pancreatitis, trauma-induced shock, bronchial asthma, cystic fibrosis, stroke, acute bronchitis, chronic bronchitis, acute bronchiolitis, chronic bronchiolitis, osteoarthritis, gout, spondyloarthropathy, ankylosing spondylitis, Reiter's syndrome, psoriatic arthropathy, enteropathic spondylitis, juvenile arthropathy, juvenile ankylosing spondylitis, reactive arthropathy, infectious arthritis, post-infectious arthritis, gonococcal arthritis, tuberculous arthritis, viral arthritis, fungal arthritis, syphilitic arthritis, Lyme disease, arthritis associated with vasculitic syndrome, polyarteritis nodosa, hypersensitivity vasculitis, Lou Gehrig's granulomatosis, rheumatic polymyalgia, articular cell arteritis, calcium crystal deposition arthropathy, pseudogout, non-articular rheumatism, bursitis, tenosynovitis, epicondylitis, neuropathic joint disease (charcot and joint), hemorrhagic arthrosis (hemarthrosis), Henoch-Schönlein purpura, hypertrophic osteoarthropathy, multicentric reticulohistiocytoma, sarcoidosis, hemochromatosis, sickle cell anemia, hemoglobinopathy, hyperlipoproteinemia, hypogammaglobulinemia, familial Mediterranean fever, Behcet's disease, systemic lupus erythematosus, relapsing fever, psoriasis, multiple sclerosis, sepsis, septic shock, multiorgan dysfunction syndrome, acute respiratory distress syndrome, chronic obstructive pulmonary disease, acute lung injury, and broncho-pulmonary dysplasia.

In yet another embodiment of the present invention, the inflammatory bowel disease may be one or more selected from the group consisting of ulcerative colitis, Crohn's disease, Behcet's disease, collagenous colitis, lymphocytic colitis, ischemic colitis, hemorrhagic rectal ulcers, ileal pouchitis, and diversion colitis.

In yet another embodiment of the present invention, the composition according to the present invention may have one or more effects selected from the group consisting of:
i) reducing intestinal epithelial barrier damage;
ii) reducing intestinal tissue infiltration of immune cells;
iii) inhibiting a reduction in the number of goblet cells;
iv) inhibiting cecal weight loss;
v) inhibiting a reduction in intestinal length;
vi) inhibiting inflammatory cytokine production;
vii) promoting anti-inflammatory cytokine production;
viii) enhancing mucus secretion by goblet cells;
ix) promoting tight junction formation in intestinal epithelial cells; and
x) increasing the number of *Bifidobacterium* in the intestine, but is not limited thereto.

In yet another embodiment of the present invention, the food composition may be a health functional food composition, but is not limited thereto.

### [Advantageous Effects]

The present invention relates to a composition for preventing, ameliorating or treating inflammatory diseases, including EVs derived from a *Roseburia* spp. strain and/or a *Bifidobacterium* spp. strain. The EVs of the present invention have been found to reduce intestinal epithelial cell barrier damage and immune cell infiltration in inflammatory bowel disease animal models, maintain the number of goblet cells, inhibit the reduction in cecal weight and intestinal length due to enteritis, and inhibit the secretion of inflammatory cytokines through changes in intestinal flora, as well as restore mucous secretion by goblet cells and the ability of intestinal epithelial cells to form tight junctions. In particular, it was also confirmed that all EVs derived from the congeneric species strains belonging to *Roseburia* spp. or *Bifidobacterium* spp. exert strong anti-inflammatory effects. In other words, the EVs derived from the strains of *Roseburia* spp. or *Bifidobacterium* spp. according to the present invention can effectively inhibit inflammation and ameliorate tissue damage or dysfunction due to inflammation, and thus they can be provided as a composition for preventing, ameliorating, and/or treating various inflammatory diseases.

### [Description of Drawings]

FIG. 1 shows ultrathin-section transmission electron microscopy (TEM) (left) and cryo-TEM (right) photographs of *Roseburia intestinalis*-derived EVs (Ri-EVs).
FIG. 2 shows the results of measuring the size and surface charge of Ri-EVs.
FIG. 3A shows the results of measuring the particle concentration of Ri-EVs extracted from *R. intestinalis.*
FIG. 3B shows the results of measuring the particle size of Ri-EVs and *Bifidobacterium longum*-derived EVs (Bl-EVs).
FIG. 4 shows a schematic diagram of an experiment of a dextran sulfate sodium salt colitis grade (DSS)-induced IBD animal model.
FIG. 5 shows a diagram illustrating Hematoxylin-Eosin (H&E) staining and Alcian blue (AB) staining of each intestinal tissue to observe the intestinal epithelial cell barrier of the control group (control), DSS-induced IBD animal model (DSS), *R*. *intestinalis* administration group (Ri-Cell), and Ri-EV administration group (Ri-EV).
FIGS. 6A to 6D show diagrams illustrating the results of measuring histological scores, the number of goblet cells (FIG. 6A), the weight of the cecum (FIG. 6B), the length of the intestine (FIG. 6C), and the disease activity index (FIG. 6D) for a pathological analysis of the intestinal tissues of the control group, the DSS-induced IBD animal model, the *R. intestinalis* administration group, and the Ri-EV administration group.
FIGS. 7A and 7B show diagrams illustrating the results of measuring the expression levels of interleukin (IL)-1β (FIG. 7A), Mucin 2 (Muc-2), and Zonula occludens-1 (ZO-1) (FIG. 7B) in the intestinal tissue of the control group, the DSS-induced IBD animal model, the *R. intestinalis* administration group, and the Ri-EV administration group in order to confirm changes in intestinal inflammation levels, mucous protein levels, and tight junctions according to the administration of *R. intestinalis* or Ri-EV.
FIGS. 8A and 8B show diagrams showing the results of measuring the intestinal microbial communities (FIG. 8A) and Shannon diversity (FIG. 8B) of the control group, the DSS-induced IBD animal model, the *R*. *intestinalis* administration group, and the Ri-EV administration group in order to confirm changes in the intestinal microbial community according to the administration of *R. intestinalis* or Ri-EV.
FIG. 9 shows a diagram illustrating the abundance of *Bifidobacterium* in the control group, the DSS-induced IBD animal model, the *R*. *intestinalis* administration group, and the Ri-EV administration group
FIG. 10 shows the amount of nitric oxide (NO) production, the expression levels of inflammatory cytokines (TNF-α, IL-6), and the expression level of an anti-inflammatory cytokine (IL-10) measured according to the treatment with EVs derived from congeneric strains of *Roseburia spp.* (*R. intestinalis* (Ri), *R. hominis* (Rho), *R. faecis* (Rfa), and *R. inulinivorans* (Rin)) after inducing an inflammatory environment by treating mouse macrophages with LPS.
FIG. 11 shows the results of confirming the inflammation inhibitory effect through the measurement of the amount of nitric oxide production according to the treatment with EVs derived from *Roseburia spp.* strains (*R. intestinalis, R. faecis, R. hominis,* and *R. inulinivorans*) and *Bifidobacterium* spp. strains (*B. bifidum, B. breve, B. lactis, B. adolescentis,* and *B. longum*) after inducing an inflammatory environment by treating a Kupffer cell-simulated *in vitro* environment with LPS.

### [Best Mode]

Hereinafter, embodiments will be described in detail to aid understanding of the present invention. However, the following embodiments are merely illustrative of the contents of the present invention, and the scope of the present invention is not limited to the following embodiments. The embodiments of the present invention are provided to more completely explain the present invention to those skilled in the art.

The present invention relates to a pharmaceutical composition for preventing or treating inflammatory diseases, including EVs derived from a *Roseburia* spp. strain and EVs derived from a *Bifidobacterium* spp. strain as an active ingredient, and it was completed by confirming that various *Roseburia* spp. strains, *Bifidobacterium* spp. strains, and/or EVs derived therefrom may exert a strong anti-inflammatory effect as well as ameliorate tissue damage or dysfunction due to inflammation. Therefore, the EVs of the present invention can be used as a composition for preventing, ameliorating, and/or treating various inflammatory diseases.

The composition according to the present invention may include one or more selected from the group consisting of a *Roseburia* spp. strain, a lysate thereof, a culture solution thereof, EVs thereof, and a mixture of these; and a *Bifidobacterium* spp. strain, a lysate thereof, a culture solution thereof, EVs thereof, and a mixture of these. In one embodiment of the present invention, a composition according to the present invention may not include a strain.

In the present invention, *"Roseburia* spp." or *"Roseburia* genus strain" includes strains that are already known in the art or that will be newly known in the future as strains belonging to the *Roseburia* genus. *Roseburia* is a Gram-positive bacterium inhabiting the human colon and is known to have an ability to decompose sugar and produce butyrate. Any strains belonging to the *Roseburia* genus are included as the *Roseburia* spp. strains according to the present invention without limitation, and they are not limited to specific types, but a *Roseburia* spp. strain may preferably be one or more selected from the group consisting of *Roseburia intestinalis, Roseburia faecis, Roseburia hominis, Roseburia inulinivorans,* and *Roseburia cecicola.* Among these, *"Roseburia intestinalis,"* which is a microorganism first isolated from human feces, is known to maintain energy homeostasis by producing metabolites in the intestine.

In the present invention, *"Bifidobacterium* spp. or *Bifidobacterium* genus strain" includes strains that are already known in the art or that will be newly known in the future as strains belonging to the *Bifidobacterium* genus. *Bifidobacterium* is a Gram-positive anaerobic bacterium and is one of the main types of bacteria constituting the gastrointestinal tract microflora of mammals. Some *Bifidobacteria* are also used as lactic acid bacteria. Any strains belonging to the *Bifidobacteria* genus are included as the *Bifidobacteria* spp. strains according to the present invention without limitation, and they are not limited to specific types, but a *Bifidobacteria* spp. strain may preferably be one or more selected from the group consisting of *Bifidobacterium longum, Bifidobacterium bifidum, Bifidobacterium breve, Bifidobacterium lactis,* and *Bifidobacterium adolescentis.* Among these, *"Bifidobacterium longum"* is also called Bifidobacterium and is known to be effective in inhibiting the growth of *Escherichia coli* and enhancing intestinal motility and bowel movements.

In the present invention, the term "strain" includes not only live cells themselves obtained from cultures such as culture media, but also any processed forms of strains known to those skilled in the art, for example, cell lysates, dried products, frozen products, etc., but is not limited thereto. In the present specification, the term "strain" may be used interchangeably with "bacteria" and "cells."

In the present invention, the term "lysate" may refer to a product obtained by crushing the cell wall of a strain by applying chemical treatment or physical force to the strain.

In the present invention, the term "culture solution" may refer to the entire medium including a strain, a metabolite thereof, and remaining nutrients, obtained by culturing the strain for a certain period of time in a medium capable of supplying nutrients so that the strain may grow and survive *in vitro.* In addition, the culture solution may mean a culture solution obtained by removing cells from a cell culture solution obtained by culturing a strain. Meanwhile, a liquid from which cells are removed from the culture solution is also referred to as "supernatant," and it may be obtained by allowing a culture solution to stand for a certain period of time to take only the liquid of the upper layer excluding the part that has settled in the lower layer or by taking the liquid in the upper portion after removing the cells through filtration or centrifuging the culture solution to remove the lower sediment. The "cell" refers to the strain itself of the present invention and includes the selected strain itself isolated from biological samples or the like or the strain isolated from a culture solution in which the strain has been cultured. The cells may be obtained by centrifuging a culture solution and taking the part that has settled in the lower layer, or they can be obtained by removing the liquid in the upper portion after allowing the culture solution to stand for a certain period of time because the cells have settled in the lower layer of the culture solution by gravity. The culture solution may be used interchangeably with "culture supernatant," "conditioned culture solution," or "conditioned medium."

The culture solution may include a culture medium itself obtained by culturing a strain, an extract thereof, a concentrate thereof, or a lyophilized product thereof; or a culture supernatant obtained by removing a strain from a culture solution, an extract thereof, a concentrate thereof, or a lyophilized product thereof, but is not limited thereto.

The culture solution may be obtained by culturing the strain of the present invention in an appropriate medium (e.g., reinforced clostridial medium (RCM) medium) at any temperature of 10 °C to 50 °C, 10 °C to 40 °C, 20 °C to 50 °C, 20 °C to 40 °C, or 30 °C to 40 °C for a certain period of time, for example, 4 to 50 hours, 4 to 40 hours, 4 to 30 hours, 4 to 20 hours, or 10 to 20 hours, but is not limited thereto. The culture medium and culture conditions for culturing the strain of the present invention may be appropriately selected or modified by those skilled in the art.

In one embodiment, the culture supernatant of the present invention may be obtained by removing the strain from the above-described strain culture solution by a method such as centrifugation, filtration, and the like.

In the present invention, the "concentrate" may be obtained by concentrating the above-described culture solution itself or a supernatant obtained from the culture solution by a method such as centrifugation, filtration, and the like.

In the present invention, the term "extract" refers to an extract from the above-described culture medium or a concentrate thereof and may include an extract, a diluent or concentrate of the extract, a dried or lyophilized product obtained by drying the extract, or a crude product or a purified product thereof, or a fraction obtained by fractionating the same.

In the present invention, the term "vesicle" refers to particles secreted from cells and released into the extracellular space and may include a number of different types such as exosomes, ectosomes, microvesicles, microparticles, and exosome-like vesicles. Extracellular vesicles (EVs) may reflect the state of the cells of origin (donor cells) secreting them, exhibit various biological activities depending on which cell they are secreted from, and play an important role in cell-to-cell interactions while transferring genetic materials and proteins between cells. In addition, cell-derived substances including the vesicles have the effects of causing diseases or stimulating immune cells to fight against diseases and helping to decompose and absorb substances that humans are unable to digest, through the metabolic process of microorganisms. The vesicles may be membrane-structured EVs, which are divided into an inside and an outside, have plasma membrane lipids, plasma membrane proteins, nucleic acids, and cytoplasmic components, and are smaller than the original cells. The term "EV" may be used interchangeably with "extracellular endoplasmic reticulum" and may be briefly referred to as "endoplasmic reticulum" or "vesicles."

In particular, the vesicles derived from Gram-positive bacteria such as the *Roseburia* spp. strain and *Bifidobacterium* spp. strain of the present invention may include peptidoglycan and lipoteichoic acid, which are components of the bacterial cell wall, in addition to proteins and nucleic acids.

In one embodiment of the present invention, the *Roseburia* spp. strain-derived EVs may have a single lipid membrane structure, but are not limited thereto.

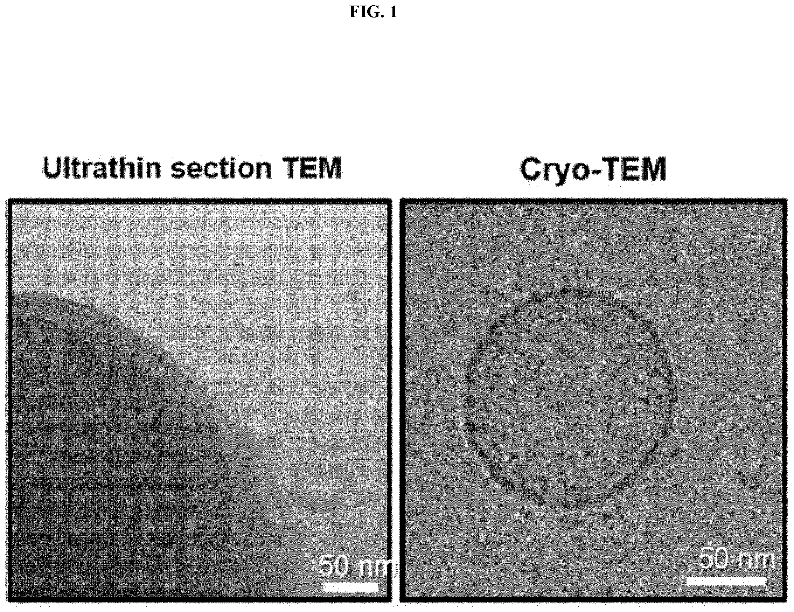

Vesicles according to the present invention may be produced during *in vitro* cultivation of strains. Specifically, the vesicles according to the present invention may be naturally secreted from the *Roseburia* spp. strain and/or *Bifidobacterium* spp. strain according to the present invention or artificially produced (isolated) through heat treatment, pressure treatment, or the like of the bacteria, and may have a diameter of 30 to 200 nm, 30 to 150 nm, 30 to 100 nm, 30 to 80 nm, 40 to 200 nm, 50 to 200 nm, 50 to 100 nm, or 50 to 80 nm. In one embodiment of the present invention, as a result of measuring the sizes of *R. intestinalis-* derived EVs and *B. longum*-derived EVs using a Zetasizer, it was confirmed that each had an average particle size of about 76 nm (FIG. 1).

In addition, the EVs according to the present invention may have a surface charge of -20 to 5 mV, -20 to 0 mV, -10 to 0 mV, -5 to 0 mV, -10 to -1 mV, or -5 to -1 mV, but are not limited thereto.

The vesicles may be isolated by applying one or more methods selected from the group consisting of centrifugation, ultra-high-speed centrifugation, extrusion, sonication, cell lysis, homogenization, freeze-thaw, electroporation, mechanical disassembly, chemical treatment, filtration with a filter, gel filtration chromatography, free-flow electrophoresis, and capillary electrophoresis to a culture solution including a *Roseburia* spp. strain and/or a *Bifidobacterium* spp. strain. In addition, processes such as washing to remove impurities and concentration of the obtained vesicles may be further included.

The composition according to the present invention may have effects of preventing, ameliorating, and/or treating inflammatory diseases.

For example, a composition according to the present invention may have one or more effects selected from among the following:
i) reducing intestinal epithelial barrier damage;
ii) reducing intestinal tissue infiltration of immune cells;
iii) inhibiting a reduction in the number of goblet cells;
iv) inhibiting cecal weight loss;
v) inhibiting a reduction in intestinal length;
vi) inhibiting inflammatory cytokine production;
vii) promoting anti-inflammatory cytokine production;
viii) enhancing mucus secretion by goblet cells;
ix) promoting tight junction formation in intestinal epithelial cells; and
x) increasing the number of *Bifidobacterium* in the intestine.

In other words, the effects of preventing, ameliorating, and/or treating inflammatory diseases of the composition according to the present invention may be exerted by the effects from i) to x).

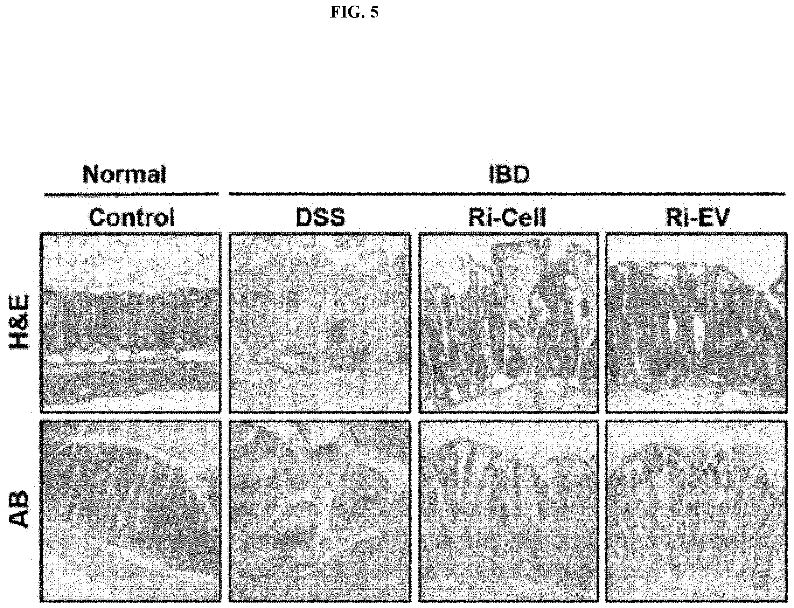

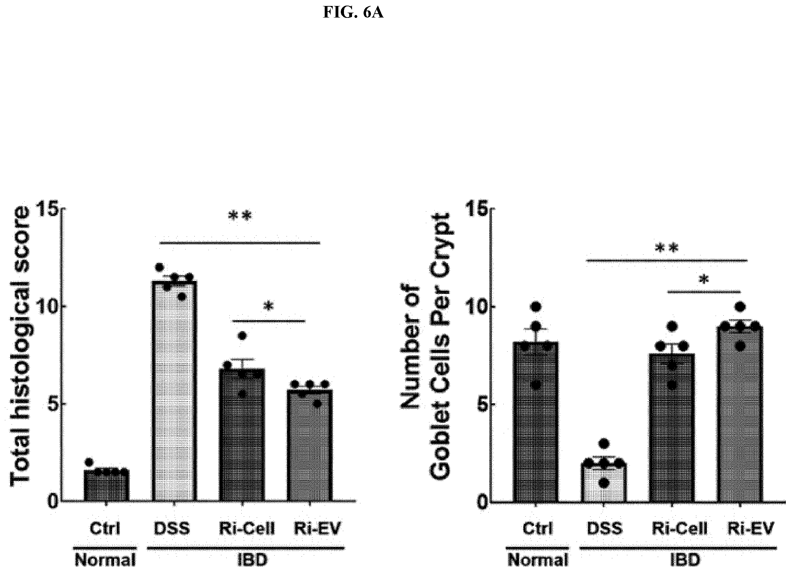

Regarding the effects of the composition of the present invention of i) reducing intestinal epithelial barrier damage; ii) reducing infiltration of immune cells; and iii) inhibiting a reduction in the number of goblet cells, as a result of experiments using an IBD animal model in one embodiment of the present invention, in the IBD animal model, the intestinal epithelial cell barrier was damaged, the immune cells were infiltrated, and the number of goblet cells responsible for mucus production was reduced compared to the normal control group, whereas in the Ri-EV administration group of the present invention, the intestinal epithelial cell barrier damage was ameliorated, immune cell infiltration was reduced, and the number of goblet cells was maintained at a level similar to that of the normal control group. In particular, it was confirmed that when the Ri-EVs were administered, since the histological score due to IBD onset significantly decreased, the intestinal damage and functional abnormalities due to IBD were ameliorated (FIGS. 5 and 6A).

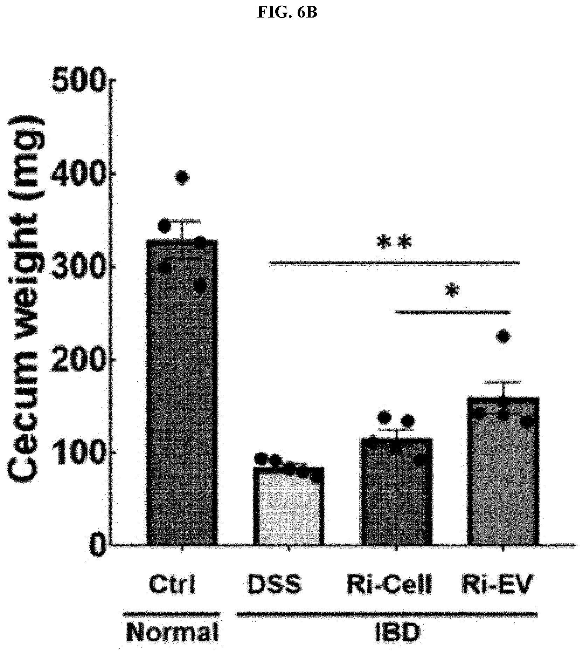

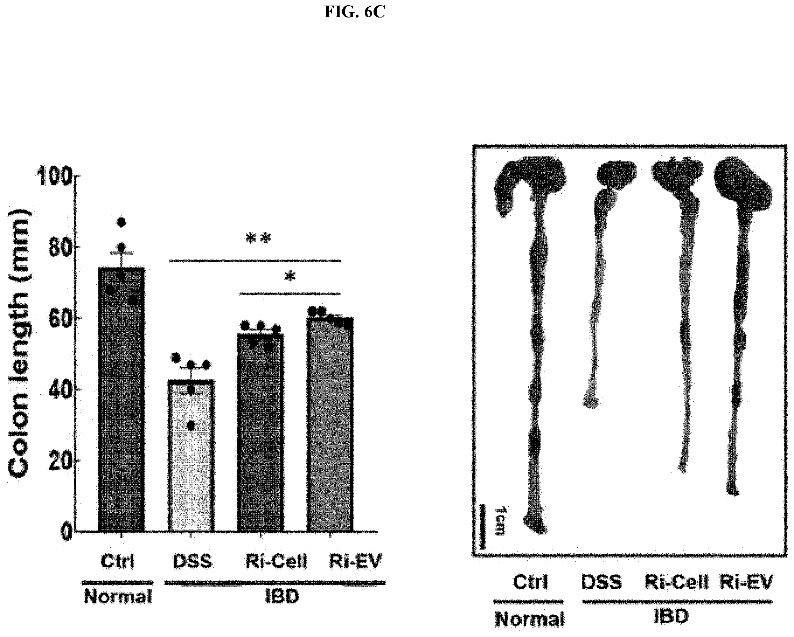

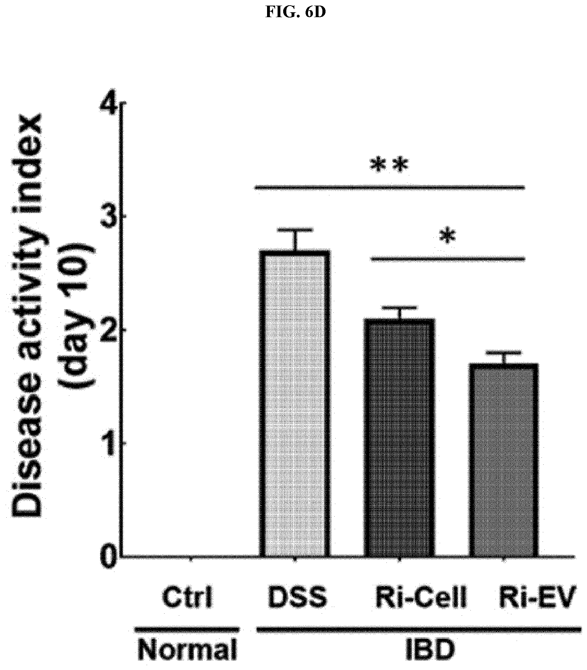

Regarding the effects of the composition of the present invention of iv) inhibiting cecal weight loss and v) inhibiting a reduction in intestinal length, in another embodiment of the present invention, the cecal weight and intestinal length were significantly reduced due to intestinal inflammation in the IBD animal model compared to the normal control group, whereas the *R. intestinalis* or Ri-EV administration group inhibited the reduction in cecal weight and intestinal length, and in particular, the cecal and intestinal repair/improvement effects of the EVs were much superior to that of the strain itself (FIGS. 6B and 6C). It was confirmed that the disease activity index also decreased when *R. intestinalis* or Ri-EVs were administered, and the disease activity was the lowest in the Ri-EV administration group (FIG. 6D). The above results show that the EVs according to the present invention may ameliorate histological changes in the cecum or intestine caused by intestinal inflammation.

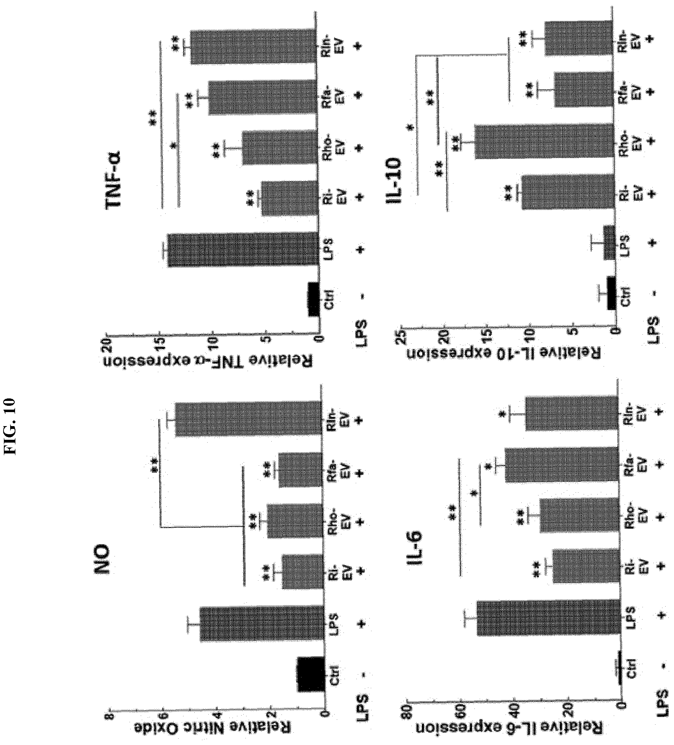

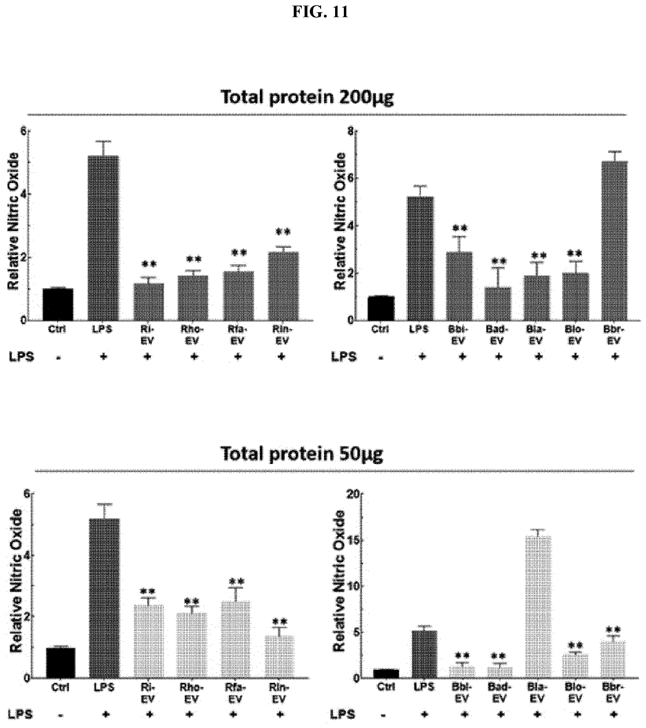

Regarding the effects of the composition of the present invention of vi) inhibiting inflammatory cytokine secretion; vii) promoting anti-inflammatory cytokine production; viii) enhancing mucus secretion by goblet cells; and ix) promoting tight junction formation in intestinal epithelial cells, in another embodiment of the present invention, it was confirmed that as the expression of the inflammatory cytokine IL-1β increased in the IBD animal model compared to the normal control group and the expression of Muc-2 and ZO-1 associated with mucous proteins and tight junctions decreased, intestinal inflammation increased, and mucus secretion and tight junctions weakened. On the other hand, in the *R. intestinalis* or Ri-EV administration group, the expression of IL-1β decreased, and the expression of Muc-2 and ZO-1 increased. In particular, it was shown that the intestinal anti-inflammatory effect and the mucus secretion ability and tight junction restoration ability were much superior in the EVs compared to the strains. In addition, as a result of isolating EVs from various congeneric *Roseburia* spp. strains and *Bifidobacterium* spp. strains and confirming their anti-inflammatory effects, it was confirmed that all the EVs inhibited the production of inflammatory factors and promoted the production of anti-inflammatory cytokines, thereby exerting excellent anti-inflammatory effects (FIGS. 10 and 11).

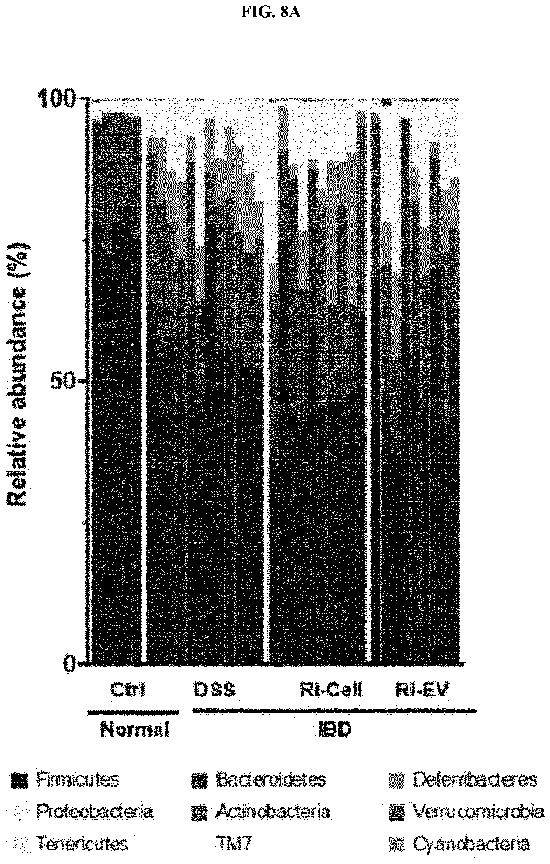

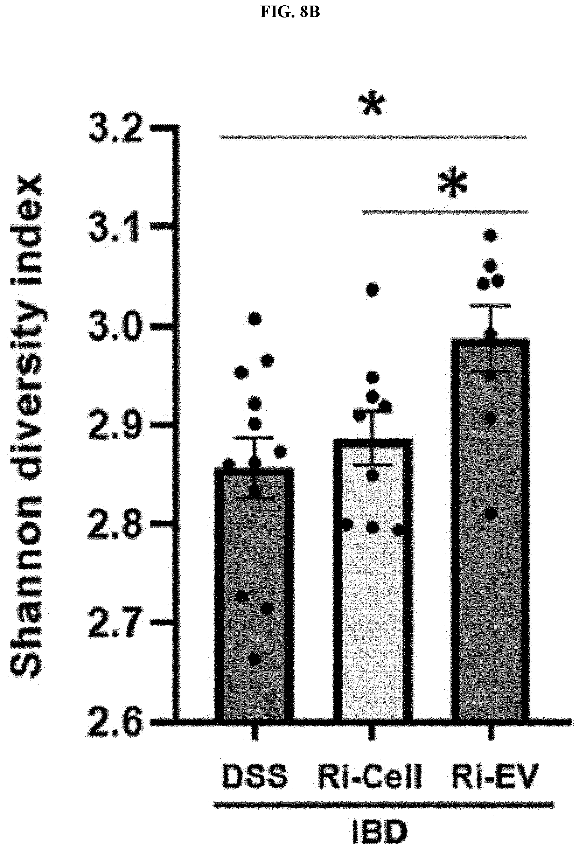

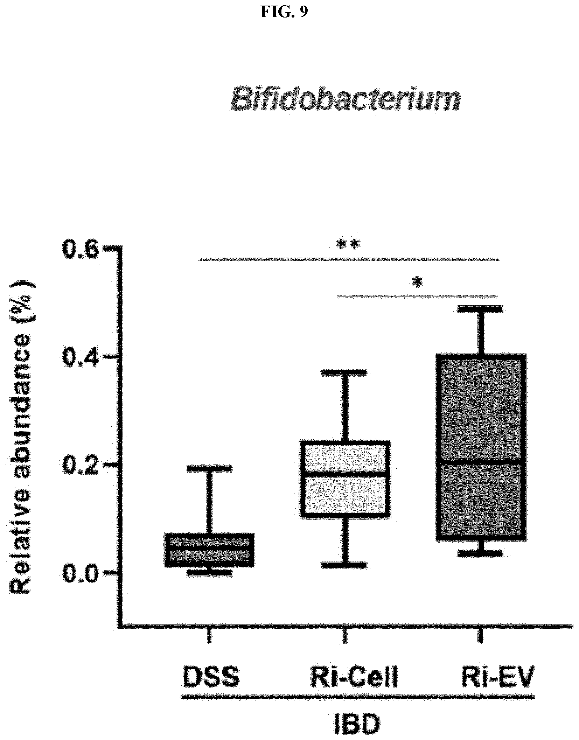

Regarding the effect of the composition of the present invention of x) increasing the number of *Bifidobacterium* in the intestine, in another embodiment of the present invention, in the IBD animal model compared to the control group, changes in the intestinal microbial community, such as an increase in the *Proteobacteria* and *Deferribacteres* phyla, were clearly observed (FIGS. 8A and 8B), whereas the number of *Bifidobacterium,* which is known to be bacteria contributing to the amelioration of IBD, increased in the *R. intestinalis* or Ri-EV administration group, and in particular, the number of *Bifidobacterium* increased significantly in the Ri-EV administration group compared to the *R. intestinalis* administration group (FIG. 9). From this, it can be seen that the administration of the *Roseburia* spp. strain-derived EVs according to the present invention induces changes in the intestinal microflora according to the growth induction of *Bifidobacterium* in the intestinal environment of IBD to inhibit the secretion of inflammatory cytokines and restore the mucus secretion ability of goblet cells and the ability of intestinal epithelial cells to form tight junctions, thereby ameliorating IBD.

In summary, the composition according to the present invention may reduce intestinal epithelial cell barrier damage due to inflammation and infiltration of immune cells into the intestine, maintain the number of goblet cells at a normal level, inhibit the decrease in cecal weight and intestinal length due to intestinal inflammation, inhibit the secretion of inflammatory cytokines through changes in intestinal microflora, and restore mucus secretion ability of goblet cells and the ability of intestinal epithelial cells to form tight junctions, and thus can be used for preventing, ameliorating, and/or treating inflammatory diseases.

Meanwhile, the composition according to the present invention may be used for preventing, ameliorating, and/or treating "inflammatory diseases." In the present invention, inflammatory diseases include without limitation any diseases related to inflammation or diseases that may be ameliorated/treated by inhibiting inflammation, and are not limited to specific types, but are preferably selected from the group consisting of gastritis, gastric ulcers, duodenal ulcers, inflammatory skin disease, allergic diseases, Crohn's disease, irritable bowel syndrome, ulcerative colitis, IBD, peritonitis, osteomyelitis, cellulitis, meningitis, encephalitis, pancreatitis, trauma-induced shock, bronchial asthma, cystic fibrosis, stroke, acute bronchitis, chronic bronchitis, acute bronchiolitis, chronic bronchiolitis, osteoarthritis, gout, spondyloarthropathy, ankylosing spondylitis, Reiter's syndrome, psoriatic arthropathy, enteropathic spondylitis, juvenile arthropathy, juvenile ankylosing spondylitis, reactive arthropathy, infectious arthritis, post-infectious arthritis, gonococcal arthritis, tuberculous arthritis, viral arthritis, fungal arthritis, syphilitic arthritis, Lyme disease, arthritis associated with vasculitic syndrome, polyarteritis nodosa, hypersensitivity vasculitis, Lou Gehrig's granulomatosis, rheumatic polymyalgia, articular cell arteritis, calcium crystal deposition arthropathy, pseudogout, non-articular rheumatism, bursitis, tenosynovitis, epicondylitis (tennis elbow), neuropathic joint disease (charcot and joint), hemorrhagic arthrosis (hemarthrosis), Henoch-Schönlein purpura, hypertrophic osteoarthropathy, multicentric reticulohistiocytoma, sarcoidosis, hemochromatosis, sickle cell anemia and other hemoglobinopathies, hyperlipoproteinemia, hypogammaglobulinemia, familial Mediterranean fever, Behcet's disease, systemic lupus erythematosus, relapsing fever, psoriasis, multiple sclerosis, sepsis, septic shock, multiorgan dysfunction syndrome, acute respiratory distress syndrome, chronic obstructive pulmonary disease, acute lung injury, and broncho-pulmonary dysplasia.

In the present invention, the term "inflammatory bowel disease (IBD)" refers to a disease caused by chronic inflammation in the gastrointestinal tract. IBD refers to a disease in which chronic inflammation occurs in the intestine and whose major symptoms are recurrent abdominal pain, diarrhea, bloody stools, weight loss, and the like. Specifically, IBD collectively refers to chronic digestive diseases in which inflammation in the intestines lasts for six months or longer and becomes chronic. Therefore, infectious enteritis such as bacterial, viral, amebic, and tuberculous enteritis, ischemic bowel disease, and radiation-induced enteritis may be included therein. In the present invention, IBD includes without limitation any diseases related to intestinal inflammation or diseases that may be ameliorated/treated by inhibiting inflammation, and is not limited to specific types, but is preferably one or more selected from the group consisting of ulcerative colitis, Crohn's disease, Behcet's disease, collagenous colitis, lymphocytic colitis, ischemic colitis, hemorrhagic rectal ulcers, ileal pouchitis, and diversion colitis.

Preferably, the IBD according to the present invention may be selected from ulcerative colitis (UC), Crohn's disease (CD), and Behcet's disease. "Ulcerative colitis" refers to a chronic inflammatory disease in the large intestine, and depending on its scope, it may be further divided into proctitis, left-sided colitis, and extensive colitis. "Crohn's disease" refers to a disease in which inflammation occurs throughout the digestive tract, from the mouth to the anus. However, the inflammatory sites are not always continuous, and they may be present separately in several places. About 1/3 of Crohn's disease patients have inflammation only in the small intestine, 1/3 of them have inflammation only in the large intestine, and the remaining 1/3 of them have chronic inflammation in both the large intestine and small intestine. In particular, the most common case is where inflammation occurs in the area where the end of the small intestine meets the large intestine. Inflammation occurs only in the mucosal layer of the intestine in ulcerative colitis, whereas Crohn's disease is characterized by an inflammatory response that invades all layers of the intestinal wall, including the mucosal, submucosal, muscular, and serosal layers. "Behcet's disease" is a chronic and repetitive systemic disease in which inflammation invades various organs such as the skin, mucous membranes, eyes, intestines, joints, genitourinary organs, and the nervous system. In 3 to 5% of Behcet's disease patients, inflammation occurs in the small or large intestine, and inflammation most commonly occurs in the area where the end of the small intestine and the large intestine are connected.

The content of the strain, lysate, culture solution, and/or EVs in the composition of the present invention may be appropriately adjusted according to the symptoms of the disease, the degree of progression of the symptoms, the conditions of the patient, etc., and for example, it may be 0.0001% to 99.9% by weight, or 0.001% to 50% by weight based on the total weight of the composition, but is not limited thereto. The content ratio is a value based on the dry amount after removing the solvent.

The pharmaceutical composition according to the present invention may further include appropriate carriers, excipients, and diluents that are commonly used in preparation of pharmaceutical compositions. The excipient may be, for example, one or more selected from the group consisting of diluents, binders, disintegrants, lubricants, adsorbents, humectants, film-coating materials, and controlled-release additives.

The pharmaceutical composition according to the present invention may be formulated in the form of powder, granules, sustained-release granules, enteric-coated granules, solutions, eye drops, elixirs, emulsions, suspensions, spirits, troches, perfumes, limonade, tablets, sustained-release tablets, enteric-coated tablets, sublingual tablets, hard capsules, soft capsules, sustained-release capsules, enteric-coated capsules, pills, tinctures, soft extracts, dry extracts, liquid extracts, injections, capsules, perfusates, plasters, lotions, pastes, sprays, inhalants, patches, sterilized injection solutions, or external preparations such as aerosols by conventional methods and used, and the external preparations may be formulated as creams, gel, patches, sprays, ointments, plasters, lotions, liniments, pastes, or cataplasmas.

Carriers, excipients, and diluents that may be included in the pharmaceutical composition according to the present invention include lactose, dextrose, sucrose, oligosaccharides, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

In the case of formulation, the composition according to the present invention may be prepared using diluents or excipients such as commonly used fillers, extenders, binders, wetting agents, disintegrants, and surfactants.

As additives for tablets, powder, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, calcium hydrogen phosphate, calcium sulfate, sodium chloride, sodium bicarbonate, purified lanolin, microcrystalline cellulose, dextrin, sodium alginate, methylcellulose, sodium carboxymethylcellulose, kaolin, urea, colloidal silica gel, hydroxypropyl starch, hydroxypropylmethyl cellulose (HPMC) 1928, HPMC 2208, HPMC 2906, HPMC 2910, propylene glycol, casein, calcium lactate, and Primogel; binders such as gelatin, gum arabic, ethanol, agar powder, cellulose acetate phthalate, carboxymethyl cellulose, calcium carboxymethyl cellulose, glucose, purified water, sodium caseinate, glycerin, stearic acid, sodium carboxymethyl cellulose, sodium methyl cellulose, methyl cellulose, microcrystalline cellulose, dextrin, hydroxycellulose, hydroxypropyl starch, hydroxymethyl cellulose, refined shellac, starch gelatin, hydroxypropyl cellulose, hydroxypropyl methylcellulose, polyvinyl alcohol, and polyvinylpyrrolidone may be used; disintegrants such as hydroxypropyl methyl cellulose, corn starch, agar powder, methyl cellulose, bentonite, hydroxypropyl starch, sodium carboxymethyl cellulose, sodium alginate, calcium carboxymethyl cellulose, calcium citrate, sodium lauryl sulfate, silicic acid anhydride, 1-hydroxypropyl cellulose, dextran, ion exchange resins, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, white sugar, magnesium aluminum silicate, D-sorbitol solution, and light anhydrous silicic acid; and lubricants such as calcium stearate, magnesium stearate, stearic acid, hydrogenated vegetable oils, talc, *Lycopodium* spores, kaolin, Vaseline, sodium stearate, cacao fat, sodium salicylate, magnesium salicylate, polyethylene glycol (PEG) 4000, PEG 6000, liquid paraffin, hydrogen-added soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oils, paraffin oils, polyethylene glycol fatty acid ether, starch, sodium chloride, sodium acetate, sodium oleate, dl-leucine, and light anhydrous silicic acid may be used.

As additives for liquid formulations according to the present invention, water, dilute hydrochloric acid, dilute sulfuric acid, sodium citrate, sucrose monostearate, polyoxyethylene sorbitol fatty acid esters (Tween esters), polyoxyethylene monoalkyl ethers, lanolin ethers, lanolin esters, acetic acid, hydrochloric acid, ammonia water, ammonium carbonate, potassium hydroxide, sodium hydroxide, prolamin, polyvinylpyrrolidone, ethyl cellulose, sodium carboxymethyl cellulose or the like may be used.

A solution of white sugar, other sugars, or sweeteners or the like may be used in the syrup according to the present invention, and flavoring agents, colorants, preservatives, stabilizers, suspending agents, emulsifiers, thickening agents or the like may be used as needed.

Purified water may be used in the emulsions according to the present invention, and emulsifiers, preservatives, stabilizers, flavoring agents or the like may be used as needed.

Suspending agents such as acacia, tragacanth, methylcellulose, carboxymethylcellulose, sodium carboxymethylcellulose, microcrystalline cellulose, sodium alginate, HPMC, HPMC 1828, HPMC 2906, HPMC 2910 or the like may be used in the suspensions according to the present invention, and surfactants, preservatives, stabilizers, colorants, and flavoring agents may be used as needed.

The injections according to the present invention may include solvents such as distilled water for injection, 0.9% sodium chloride for injection, Ringer's solution, dextrose for injection, dextrose + sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid, benzyl benzoate; solubilizing agents such as sodium benzoate, sodium salicylate, sodium acetate, urea, urethane, monoethyl acetamide, butazolidine, propylene glycol, Tweens, nicotinic acid amide, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediaminetetraacetic acid; antioxidants agents such as sodium bisulfide 0.1%, sodium formaldehyde sulfoxylate, thiourea, disodium ethylenediaminetetraacetate, and acetone sodium bisulfite; analgesics such as benzyl alcohol, chlorobutanol, procaine hydrochloride, glucose, and calcium gluconate; and suspending agents such as carboxymethyl cellulose (CMC) sodium, sodium alginate, Tween 80, and aluminum monostearate.

In the suppositories according to the present invention, bases such as cacao fat, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, Hexalide Base 95, Cotomar, Hydrocote SP, S-70-XXA, S-70-XX75 (S-70-XX95), Hydrokote 25, Hydrokote 711, Idropostal, Massa estrarium (A, AS, B, C, D, E, I, T), Massa-MF, Massaupol, Masupol-15, Neosupostal-N, Paramount-B, Suposiro (OSI, OSIX, A, B, C, D, H, L), suppositories base type IV (AB, B, A, BC, BBG, E, BGF, C, D, 299), Supostal (N, Es), Wecobi (W, R, S, M, Fs), Tegester triglyceride base (TG-95, MA, 57) may be used.

Solid preparations for oral administration include tablets, pills, powder, granules, and capsules. These solid preparations are prepared by mixing the extract with at least one excipient, for example, starch, calcium carbonate, sucrose, lactose, gelatin, etc. In addition to simple excipients, lubricants such as magnesium stearate and talc are also used.

Suspensions, oral solutions, emulsions, syrup, and the like correspond to liquid preparations for oral administration, and in addition to the commonly used simple diluents such as water and liquid paraffin, various excipients, for example, wetting agents, sweeteners, flavoring agents, and preservatives may be included. Preparations for parenteral administration include sterilized aqueous solutions, non-aqueous solutions, suspensions, emulsions, freeze-dried preparations, and suppositories. As non-aqueous solvents and suspensions, propylene glycol, polyethylene glycol, vegetable oils such as olive oil, and an injectable ester such as ethyl oleate may be used.

The pharmaceutical composition according to the present invention is administered in a pharmaceutically effective amount. In the present invention, "pharmaceutically effective amount" refers to an amount that is sufficient to treat a disease with a reasonable benefit/risk ratio applicable to medical treatment, and an effective dose level may be determined based on factors including the type and severity of a patient's disease, drug activity, sensitivity to the drug, administration time, administration route and excretion rate, treatment duration, and concurrently used drugs, and other factors well known in the medical field.

The pharmaceutical composition according to the present invention may be administered as an individual therapeutic agent or in combination with another therapeutic agent, and it may be administered sequentially or simultaneously with conventional therapeutic agents, and it may be administered once or multiple times. It is important to administer an amount that may achieve the maximum effect with the minimum amount without side effects by considering all of the above factors, and this may be easily determined by those skilled in the art to which the present invention pertains.

The pharmaceutical composition of the present invention may be administered to a subject through various routes. All modes of administration may be considered, and it may be administered by, for example, oral administration, subcutaneous injection, intraperitoneal administration, intravenous injection, intramuscular injection, paraspinal space (intrathecal) injection, sublingual administration, buccal administration, intrarectal injection, vaginal injection, ocular administration, auricular administration, nasal administration, inhalation, spraying through the mouth or nose, dermal administration, transdermal administration or the like.

The pharmaceutical composition of the present invention is determined according to the type of drug that is an active ingredient along with various relevant factors such as the disease to be treated, administration route, the patient's age, gender, and weight, and the severity of the disease. Specifically, the effective amount of the composition according to the present invention may vary depending on the patient's age, sex, and weight, and is generally 0.001 to 150 mg, preferably 0.01 to 100 mg per 1 kg of body weight, and may be administered daily or every other day, or 1 to 3 times a day. However, since it may increase or decrease depending on the administration route, severity of disease, sex, weight, age, etc., the dosage does not limit the scope of the present invention in any way.

In the present invention, "subject" refers to a subject in need of treatment of a disease, and more specifically, human or non-human primates, and mammals such as mice, rats, dogs, cats, horses, and cows.

In the present invention, "administration" refers to providing a predetermined amount of the composition of the present invention to a subject by any appropriate method.

In the present invention, "prevention" refers to all actions that suppress or delay the onset of a target disease, "treatment" refers to all actions that improve or beneficially change a target disease and associated metabolic abnormalities thereof by administration of the pharmaceutical composition according to the present invention, and "amelioration" refers to all actions that reduce parameters related to a target disease, for examples, the degree of symptoms, by administration of the pharmaceutical composition according to the present invention

In addition, the present invention provides a kit for preventing, ameliorating, or treating inflammatory diseases, including the composition according to the present invention.

The kit according to the present invention is not limited to a specific form as long as it may achieve the purpose of preventing, ameliorating, or treating inflammatory diseases, and it may include without limitation any components and devices for the preparation (e.g., culturing of strains, isolation of EVs, etc.), storage, administration, etc. of the composition according to the present invention. In addition, the kit may include a manual containing general descriptions of the composition of the present invention, such as the properties of the composition, methods of using it, methods of storing it, and the dosage.

In addition, the present invention provides a food composition for preventing or ameliorating inflammatory diseases, including one or more selected from the group consisting of EVs derived from a *Roseburia* spp. strain and EVs derived from a *Bifidobacterium* spp. strain as an active ingredient. The food composition includes a health functional food composition.

When the strain, lysate, culture solution, and/or EVs of the present invention are used as a food additive, the strain, EVs, and the like may be added as is or used together with other food or food ingredients, and may be appropriately used according to conventional methods. The content of the active ingredient in the food composition may be appropriately determined depending on the purpose of use (prevention, amelioration or therapeutic treatment). The mixing amount of the active ingredient may be appropriately determined according to the purpose of use (prevention, improvement or therapeutic treatment). In general, when preparing food or beverages, the strain, EVs, and the like of the present invention may be added in an amount of 15% by weight or less, or 10% by weight or less based on the raw materials. However, in the case of long-term intake for the purpose of health and hygiene or health control, the amount may be less than the above range, and since there is no problem in terms of safety, the active ingredient may also be used in an amount greater than the above range.

There is no particular limitation on the type of food. Examples of food to which the above substances may be added include meat, sausages, bread, chocolates, candies, snacks, confectioneries, pizza, ramen, other noodles, gum, dairy products including ice cream, various soups, beverages, tea, drinks, alcoholic beverages, and vitamin complexes, and include all health functional foods in a conventional sense.

The health beverage composition according to the present invention may contain various flavoring agents or natural carbohydrates as additional ingredients, like conventional beverages. The above-described natural carbohydrates are monosaccharides such as glucose and fructose, disaccharides such as maltose and sucrose, polysaccharides such as dextrin and cyclodextrin, and sugar alcohols such as xylitol, sorbitol, and erythritol. As a sweetener, natural sweeteners such as thaumatin and stevia extract, or synthetic sweeteners such as saccharin and aspartame may be used. The proportion of the natural carbohydrates is generally about 0.01 to 0.20 g, or about 0.04 to 0.10 g per 100 mL of the composition of the present invention.

In addition to the above, the composition of the present invention may contain various nutrients, vitamins, electrolytes, flavoring agents, colorants, pectic acid and salts thereof, alginic acid and salts thereof, organic acids, protective colloidal thickeners, pH regulators, stabilizers, preservatives, glycerin, alcohols, carbonating agents used in carbonated beverages, and the like. In addition, the composition of the present invention may contain fruit flesh for preparing natural fruit juice, fruit juice beverages, and vegetable beverages. These ingredients may be used independently or in combination. The proportion of these additives is not important, but is generally selected in the range of 0.01 to 0.20 parts by weight per 100 parts by weight of the composition of the present invention.

In the present specification, "health functional food" is the same term as food for special health use (FoSHU), and refers to food with a high medicinal and healthcare effect that is processed to efficiently exhibit bioregulatory functions in addition to supplying nutrients. To obtain useful effects for preventing or ameliorating inflammatory diseases, the food may be prepared in various forms such as tablets, capsules, powder, granules, liquids, and pills.

The health functional food of the present invention may be prepared by a method commonly used in the art and may be prepared by adding raw materials and ingredients commonly added in the art during preparation. In addition, unlike general drugs, there is an advantage in that the health functional food has no side effects that may occur when taking a drug for a long time by using food as a raw material, and it may have excellent portability.

In addition, the present invention provides a feed composition for preventing or ameliorating inflammatory diseases, including one or more selected from the group consisting of EVs derived from a *Roseburia* spp. strain and EVs derived from a *Bifidobacterium* spp. strain as an active ingredient.

The term "feed" of the present invention refers to any natural or artificial diet, one-meal diet, or ingredients of the one-meal diet suitable for animals to eat, ingest, and digest. The feed containing the graphene nanoparticles of the present invention as an active ingredient may be prepared from various types of feed known in the art and may specifically include concentrate feed, roughage, and/or special feed.

The strain, lysate, culture solution, and/or EVs of the present invention included in the feed composition of the present invention may vary depending on the purpose and conditions of use of the feed, and for example, it may be 0.01% to 100% by weight, more specifically, 1% to 80% by weight based on the total weight of the livestock feed composition, but is not limited thereto.

When the composition is prepared as a feed additive, the composition may be prepared as a highly concentrated product of 20% to 90% or in powder or granular form. The feed additive may further include any one or more of organic acids such as citric acid, fumaric acid, adipic acid, lactic acid, and malic acid, phosphates such as sodium phosphate, potassium phosphate, acid pyrophosphate, and polyphosphate (polymeric phosphate), and natural antioxidants such as polyphenols, catechin, alpha-tocopherol, rosemary extract, vitamin C, green tea extract, licorice extract, chitosan, tannic acid, and phytic acid. When prepared as feed, the composition may be formulated in a conventional feed form and may include conventional feed ingredients together.

The feed and feed additives may further include grains such as milled or ground wheat, oat, barley, corn, and rice; vegetable protein feed such as feed based on rapeseed, soybean, and sunflower; animal protein feed such as blood meal, meat meal, bone meal, and fish meal; sugars and dairy products such as dry ingredients composed of various types of powdered milk and whey powder, and they may further include nutritional supplements, digestion and absorption enhancers, growth promoters, and the like.

The feed additive may be administered to animals independently or in combination with other feed additives in an edible carrier. In addition, the feed additives may be easily administered to animals as a top dressing, after being directly mixed with animal feed, or in an oral formulation separate from feed. When the feed additive is administered separately from animal feed, it may be prepared as an immediate release or sustained release formulation by combining it with a pharmaceutically acceptable edible carrier, as is well known in the art. Such edible carriers may be solid or liquid, and they may be, for example, corn starch, lactose, sucrose, soybean flakes, peanut oil, olive oil, sesame oil, and propylene glycol. When a solid carrier is used, the feed additive may be tablets, capsules, powder, troches or sugar-containing tablets or a top dressing in a microdispersible form. When a liquid carrier is used, the feed additive may be in the form of gelatin soft capsules, or syrups, suspensions, emulsions, or solutions.

In addition, the feed and feed additives may contain auxiliary agents, such as preservatives, stabilizers, wetting agents or emulsifying agents, solution accelerators, and the like. The feed additive may be used by adding it to an animal's feed by steeping, spraying or mixing.

The feed or feed additive of the present invention may be applied to the diet of a number of animals including mammals, poultry, and fish.

As the mammals, it may be used not only for pigs, cows, horses, sheep, rabbits, goats, rodents, and laboratory rodents such as rats, hamsters, and guinea pigs, but also for companion animals (e.g., dogs and cats). As the poultry, it may also be used for chickens, turkeys, ducks, geese, pheasants, and quails, and as the fish, it may be used for carp, crucian carp, and trout, but is not limited thereto.

Hereinafter, examples will be described in detail to aid understanding of the present invention. However, the following examples are merely illustrative of the contents of the present invention, and the scope of the present invention is not limited to the following examples. The examples of the present invention are provided to more completely explain the present invention to those skilled in the art.

### [Examples]

### Example 1. Extraction and size analysis of EVs derived from Roseburia intestinalis or Bifidobacterium longum (Ri-EVs, Bl-EVs)

To extract EVs from each of *R. intestinalis* and *B. longum, R. intestinalis* KCTC 15746 and *B. longum* KCTC 3249 were cultured for 18 hours in an anaerobic chamber at 37 °C using reinforced clostridial medium (RCM) (MBcell, MB-R1602). After centrifuging the culture solution at 2000×g for 20 minutes, the supernatant was collected, and cell debris and waste products in the culture medium were removed using a 0.22 µm filter. EVs were extracted from the culture solution through a 300 kDa molecular weight Tangential Flow Filtration (TFF) membrane filter-based TFF system, diluted in phosphate-buffered saline (PBS), and purified, and finally *R. intestinalis*-derived EVs (Ri-EVs) and *B. longum*-derived EVs (Bl-EVs) dispersed in PBS were extracted.

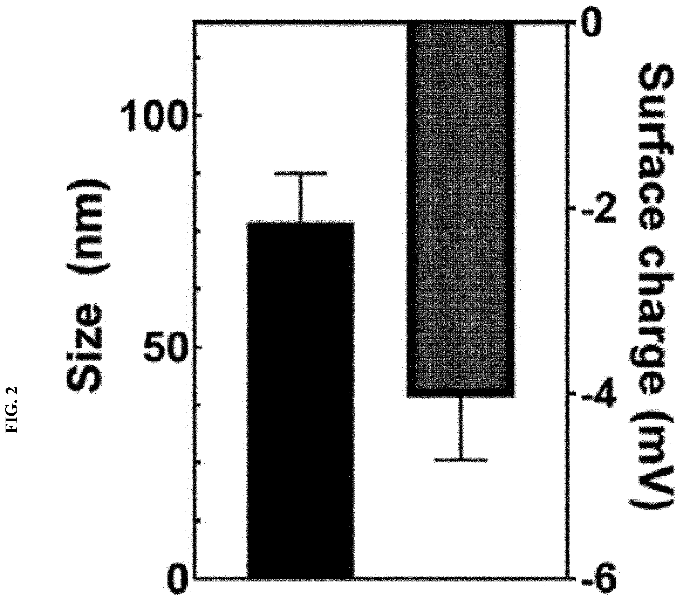

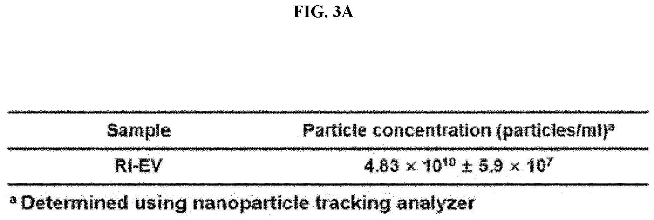

Next, the physicochemical properties of Ri-EVs were confirmed through an electron microscope (ultrathin-section transmission electron microscopy (TEM), cryo-TEM) and a nanoparticle tracking analyzer. As a result, it was confirmed through ultrathin-section TEM that *R. intestinalis* produced EVs during *in vitro* culture, and it was confirmed through cryo-TEM that the EVs had a single lipid membrane structure (FIG. 1). The extracted Ri-EVs had a size of 76 ± 10 nm and a surface charge of -4 ± 1 mV (FIG. 2) and were extracted at a concentration of 4.8 × 10¹⁰ ± 5.9×10⁷ particles/ml (FIG. 3A).

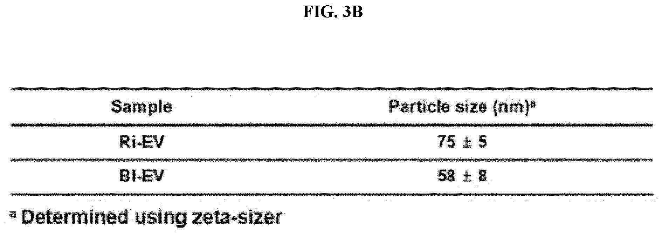

In addition, as a result of measuring the sizes of Ri-EVs and Bl-EVs using a Zetasizer, it was confirmed that they had an average particle size of 75 nm and 58 nm, respectively (FIG. 3B).

### Example 2. Evaluation of anti-inflammatory effect of Ri-EV administration to dextran sulfate sodium salt colitis (DSS) grade-induced inflammatory bowel disease (IBD) animal model

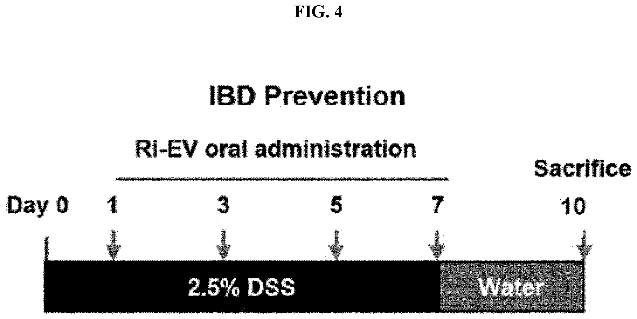

An IBD animal model was produced by providing normal C57BL/6 mice with drinking water containing DSS, which is known to cause IBD, at a concentration of 2.5% [v/v] for one week (FIG. 4). The *R. intestinalis* strain or Ri-EVs extracted in Example 1 were orally administered to the experimental groups based on a total protein of 200 µg/mouse/day on days 1, 3, 5, and 7 during the week of DSS injection. On day 10 after the start of the experiment, the mice were sacrificed to confirm the anti-inflammatory effect of the EV administration. Normal mice that were not administered DSS were used as a control group.

First, for pathological analysis of the intestinal tissue, the intestines of the mice were extracted, fixed in 4% neutral buffered formalin, and embedded in paraffin, and tissue sections having a thickness of 4 µm were prepared. After performing H&E staining and AB staining on the tissue sections, the tissue sections were observed under an optical microscope.

Next, the cecum weight and intestinal length related to intestinal inflammation were measured.

Next, to confirm the ability of Ri-EVs to regulate inflammation, mucus proteins, and tight junctions in the intestinal environment, the expression levels of IL-1β, Muc-2, and ZO-1 in the previously obtained intestinal tissue were confirmed by qRT-PCR. After dissolving the intestinal tissue in a Trizol solution (Life Technologies, CA, USA), total RNA was isolated from the tissue using the chloroform-isopropanol method according to the manufacturer's analysis method. The cDNA of the isolated sample was synthesized using a high-capacity cDNA synthesis kit (Invitrogen, CA, USA) and amplified using TaqMan gene-specific probes (Thermo Fisher Scientific) with a 7500 Real-Time PCR System (Applied Biosystems, CA, USA).

As a result, as shown in FIG. 5, the intestinal epithelial cell barrier was damaged, immune cells were infiltrated, and the number of goblet cells responsible for mucus production was reduced in the DSS-induced IBD animal model compared to the control group which was not administered DSS. On the other hand, in the Ri-EV administration group, intestinal epithelial cell barrier damage and immune cell infiltration were reduced, and the number of goblet cells was maintained at a level similar to that of the normal group. In particular, since the histological score due to the onset of IBD was significantly reduced in the Ri-EV administration group, it was confirmed that IBD was ameliorated (FIG. 6A).

In addition, the cecal weight and intestinal length related to intestinal inflammation were significantly reduced in the DSS-induced IBD animal model compared to the control group, whereas the reduction in the cecal weight and intestinal length was inhibited in the *R. intestinalis* or Ri-EV administration group. In particular, the reduction in the cecal weight and intestinal length was significantly inhibited in the Ri-EV administration group compared to *the R. intestinalis* administration group (FIGS. 6B and 6C). It was also confirmed that the disease activity index was the lowest in the Ri-EV administration group (FIG. 6D).

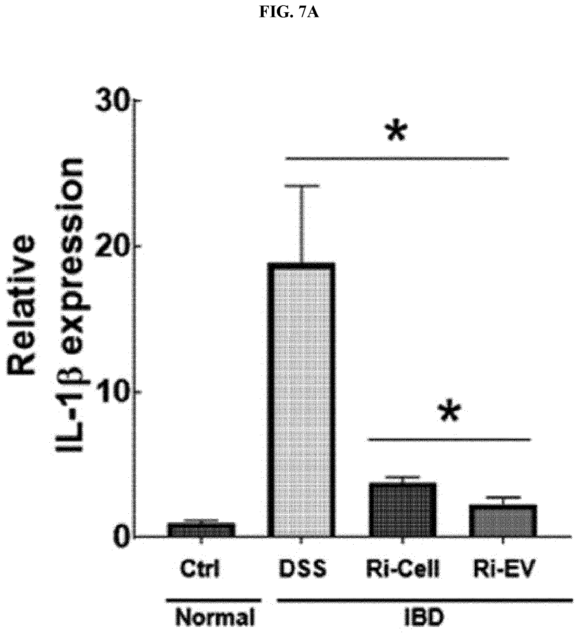

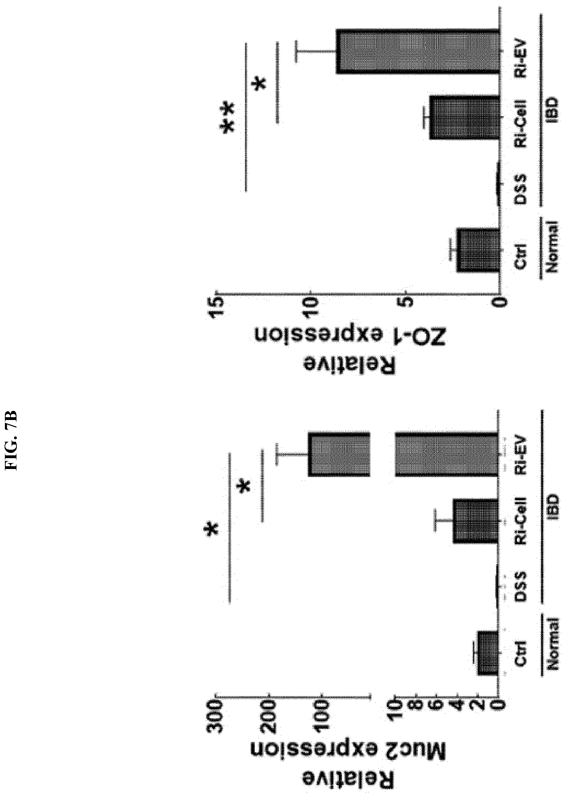

In addition, it was confirmed that the expression of IL-1β, an inflammatory cytokine that induces inflammation, increased, and the expression of Muc-2 and ZO-1 related to mucus proteins and tight junctions decreased in the DSS-induced IBD animal model compared to the control group, which increases intestinal inflammation and weakens mucus secretion and tight junctions. On the other hand, the *R. intestinalis* or Ri-EV administration group showed a decrease in the expression of IL-1β (FIG. 7A) and an increase in the expression of Muc-2 and ZO-1 (FIG. 7B) compared to the IBD animal model. In particular, the Ri-EV administration group showed a significant decrease in the expression of IL-1β and an increase in the expression of Muc-2 and ZO-1 compared to the *R*. *intestinalis* administration group. The above results indicate that Ri-EV administration can effectively reduce intestinal inflammation and restore mucus secretion ability and tight junctions, and that these effects are better than when administering the strain itself.

### Example 3. Evaluation of intestinal microflora changes according to Ri-EV administration in DSS-induced IBD animal model

To confirm the changes in the intestinal microflora and the resulting anti-inflammatory effect according to Ri-EV administration in the IBD animal model, a 16S ribosomal RNA gene sequencing analysis was performed using the QIAamp Power Fecal Pro DNA Kit (QIAGEN, Germany) with fecal samples obtained from the DDS-induced IBD animal model of Example 2.

As a result, as shown in FIG. 8A, changes in the intestinal microbial community, such as an increase in the *Proteobacteria* and *Deferribacteres* phyla, were clearly observed in the DSS-induced IBD animal model compared to the control group that was not administered DSS. In addition, it was confirmed that the Shannon diversity value increased significantly in the Ri-EV administration group compared to the other groups, indicating that various changes in the microflora occurred in the samples (FIG. 8B). In particular, as shown in FIG. 9, among the changes in the intestinal bacteria, the number of *Bifidobacterium,* which is known to be bacteria contributing to the amelioration of IBD, increased in the *R. intestinalis* or Ri-EV administration group, and the increase was significant in the Ri-EV administration group compared to the *R. intestinalis* administration group. Through this, it can be seen that Ri-EV administration ameliorates IBD by inhibiting the secretion of inflammatory cytokines through changes in the intestinal microflora caused by the induction of growth of *Bifidobacterium* in the intestinal environment of IBD and restoring mucus secretion by goblet cells and the ability of intestinal epithelial cells to form tight junctions.

### Example 4. Analysis of anti-inflammatory activity of Roseburia spp. strain-derived EVs

In this Example, it was confirmed whether EVs of other strains of the *Roseburia* spp., in addition to the EVs of *R. intestinalis,* extracted through the process of Example 1, could exert a therapeutic effect on IBD.

Specifically, in addition to *R. intestinalis* (Ri), EVs were extracted from each of three strains of the *Roseburia* spp. (*Roseburia faecis* (Rfa, KCTC 15739), *Roseburia hominis* (Rho, KCTC 5845), *Roseburia inulinivorans* (Rin, DSM 16841)), and then their anti-inflammatory activity was analyzed. The EVs were extracted in the same manner as in Example 1.

To evaluate the anti-inflammatory activity of the EVs from each strain, the amount of nitric oxide (NO) production, which is an inflammatory indicator, was measured, and the expression levels of inflammatory cytokines and anti-inflammatory cytokines were also measured, and the results are shown in FIG. 10.

Specifically, the amount of NO production was measured using mouse macrophages. Mouse macrophage Raw 264.7 cells were cultured at 37 °C in the presence of 5% CO₂ in an RPMI1640 culture solution containing 10% fetal bovine serum (FBS) and 1% antibiotics (100 U/ml penicillin and 100 µg/ml streptomycin). Thereafter, 1 ml of the culture solution was dispensed into a 12-well plate at a concentration of 5×10⁵ cells/well and cultured at 37 °C for 24 hours in a CO₂ incubator. To induce inflammation, the wells were treated with a medium supplemented with 10 µg/ml of LPS, and culture was performed for four additional hours. Then, the cells were treated by adding 2 mg of vesicles, based on the total protein amount, to the medium and then cultured at 37 °C for 16 hours. Thereafter, 50 µl of the well supernatant and 50 µl of the Griess reagent were mixed and allowed to react at room temperature for ten minutes, and then absorbance was measured at 540 nm using a plate reader to confirm the amount of NO production.

The expression levels of inflammatory cytokines and anti-inflammatory cytokines were also measured using LPS-treated Raw264.7 cells. The Raw264.7 cells treated with LPS in the same manner as above were treated with 2 mg of the vesicles based on the total protein amount, and then cultured at 37 °C for 16 hours. Thereafter, the protein expression levels of TNF-α, IL-6, and IL-10 in the cells were analyzed by measuring absorbance at 450 nm using an enzyme-linked immunosorbent assay (ELISA) kit (R&D Systems, US) according to the manufacturer's protocol.

As a result, as shown in FIG. 10, it was confirmed that when the inflammation-induced mouse macrophages were treated with congeneric *Roseburia* strain-derived EVs, the amount of NO production was significantly reduced the *Roseburia* spp. strain-derived extracellular vesicles except for Rin-derived EVs (Rin-EV). In addition, it was confirmed that in the case of the expression levels of inflammatory cytokines and anti-inflammatory cytokines, all of the EVs derived from the *Roseburia* spp. strains used as experimental groups (hereinafter, Ri-EV, Rho-EV, Rfa-EV, and Rin-EV) not only significantly and effectively reduced TNF-α and IL-6 but also increased IL-10 compared to the control group.

Through the analysis of the anti-inflammatory activity of the congeneric *Roseburia* strains, it was confirmed that the EVs derived from all the *Roseburia* spp. strains used in the experiment exhibited excellent anti-inflammatory effects, and therefore they could be used as a composition for preventing, ameliorating or treating IBD.

### Example 5. Analysis of anti-inflammatory activity of EVs derived from Roseburia spp. and Bifidobacterium spp. strains

Following Example 4, it was confirmed whether EVs derived from strains belonging to *R. intestinalis* spp. or *B. longum* spp. could exert an anti-inflammatory effect.

Specifically, EVs were extracted from each of four strains of *Roseburia* spp. (*Roseburia faecis,* (KCTC 15739), *Roseburia hominis* (KCTC 5845), *Roseburia inulinivorans* (DSM 16841)) and each of five strains of *Bifidobacterium* spp. (*Bifidobacterium bifidum* (KCTC 3281), *Bifidobacterium breve* (KCTC 3220), *Bifidobacterium lactis* (KCTC 5854), *Bifidobacterium adolescentis* (KCTC 3216)), and their anti-inflammatory activity was analyzed. The EVs were extracted in the same manner as in Example 1.

To evaluate the anti-inflammatory activity of the EVs from each strain, an *in vitro* environment simulating Kupffer cells, one type of cells constituting liver tissue, was produced using mouse macrophages Raw264.7 cells and the results of measuring the amount of NO produced by them are shown in FIG. 11. Specifically, mouse macrophage Raw 264.7 cells were cultured at 37 °C in the presence of 5% CO₂ in an RPMI1640 culture solution containing 10% FBS and 1% antibiotics (100 U/ml penicillin and 100 µg/ml streptomycin). Thereafter, 1 ml of the culture solution was dispensed into a 12-well plate at a concentration of 5×10⁵ cells/well and cultured at 37 °C for 24 hours in a CO₂ incubator. To induce inflammation, the wells were treated with a medium supplemented with 10 µg/ml of LPS, and culture was performed for four additional hours. Then, the vesicles were added to the medium at a concentration of 200 µg based on the total protein amount, and culture was performed at 37 °C for 16 hours. Thereafter, 50 µl of the well supernatant and 50 µl of the Griess reagent were mixed and allowed to react at room temperature for ten minutes, and then absorbance was measured at 540 nm using a plate reader to confirm the amount of NO production.

As a result, as shown in FIG. 11, it was confirmed that NO production by LPS was significantly and effectively reduced in all groups administered the EVs derived from the *Roseburia* spp. strains used as experimental groups. It was confirmed that NO production was effectively reduced also in the groups administered the EVs derived from the *Bifidobacterium* spp. strains, and among them, the ability of Bbr-EV and Bla-EV to inhibit NO production was controlled at a total protein amount of 200 µg or 50 µg.

The above results show that EVs of congeneric strains belonging to the *Roseburia* spp. or *Bifidobacterium* spp. exert excellent anti-inflammatory effects. In other words, from the above results, it was confirmed that EVs derived from *Roseburia* spp. and *Bifidobacterium* spp. may be used for preventing, ameliorating, or treating various inflammatory diseases through their strong anti-inflammatory effects.

As described in the above examples, it was found that the EVs derived from *Roseburia* spp. strains or *Bifidobacterium* spp. strains of the present invention reduce the intestinal epithelial barrier damage due to inflammation, reduce the infiltration of immune cells, maintain the number of goblet cells, inhibit the reduction in cecal weight and intestinal length due to enteritis, inhibit the secretion of inflammatory cytokines through changes in intestinal microflora, and restore mucus secretion by goblet cells and the ability of intestinal epithelial cells to form tight junctions. In particular, it was confirmed that all the EVs derived from various congeneric strains of *Roseburia* spp. or *Bifidobacterium* spp. exhibit excellent anti-inflammatory effects. Therefore, the EVs derived from *Roseburia* spp. strains or *Bifidobacterium* spp. strains of the present invention not only exert a strong anticancer effect but also ameliorate histological damage or loss of function due to inflammation, and thus can be provided as a composition for preventing, ameliorating, and/or treating various inflammatory diseases.

From the above description, those skilled in the art to which the present invention pertains will be able to understand that the present invention may be implemented in other specific forms without changing its technical spirit or essential features. In this regard, it should be understood that the above-described embodiments are illustrative in all respects and not restrictive. The scope of the present invention should be construed as including all changes or modifications derived from the meaning and scope of the claims to be described below and equivalent concepts thereof, rather than the above detailed description.

### [Industrial Applicability]

The present invention relates to a composition for preventing, ameliorating or treating inflammatory diseases, including EVs derived from a *Roseburia* spp. and/or *Bifidobacterium* spp. strain. It was found that the EVs of the present invention reduce the intestinal epithelial barrier damage and the infiltration of immune cells, maintain the number of goblet cells, inhibit the reduction in cecal weight and intestinal length due to enteritis, inhibit the secretion of inflammatory cytokines through changes in intestinal microflora, and restore mucus secretion by goblet cells and the tight junction formation ability of intestinal epithelial cells in an IBD animal model. In particular, it was also confirmed that the EVs derived from all congeneric strains belonging to *Roseburia* spp. strains or *Bifidobacterium* spp. strains exhibit excellent anti-inflammatory effects. In other words, the EVs derived from *Roseburia* spp. strains or *Bifidobacterium* spp. strains of the present invention may effectively inhibit inflammation and ameliorate tissue damage or dysfunction due to inflammation, and thus can be provided as a composition for preventing, ameliorating, and/or treating various inflammatory diseases.

## Claims

1. A pharmaceutical composition for preventing or treating inflammatory diseases, comprising one or more selected from the group consisting of extracellular vesicles (EVs) derived from a *Roseburia* spp. strain and EVs derived from a *Bifidobacterium* spp. strain as an active ingredient.

2. The pharmaceutical composition of claim 1, wherein the EVs have an average diameter of 30 nm to 200 nm.

3. The pharmaceutical composition of claim 1, wherein the EVs derived from a *Roseburia* spp. strain are naturally or artificially secreted from a *Roseburia* spp. strain; and the EVs derived from a *Bifidobacterium* spp. strain are naturally or artificially secreted from a *Bifidobacterium* spp. strain.

4. The pharmaceutical composition of claim 1, wherein the *Roseburia* spp. strain is one or more selected from the group consisting of *Roseburia intestinalis, Roseburia faecis, Roseburia hominis,* and *Roseburia inulinivorans.*

5. The pharmaceutical composition of claim 1, wherein the *Bifidobacterium* spp. strain is one or more selected from the group consisting of *Bifidobacterium longum, Bifidobacterium bifidum, Bifidobacterium breve, Bifidobacterium lactis,* and *Bifidobacterium adolescentis.*

6. The pharmaceutical composition of claim 1, wherein the inflammatory disease is one or more selected from the group consisting of gastritis, gastric ulcers, duodenal ulcers, inflammatory skin disease, allergic diseases, irritable bowel syndrome, ulcerative colitis, inflammatory bowel disease, peritonitis, osteomyelitis, cellulitis, meningitis, encephalitis, pancreatitis, trauma-induced shock, bronchial asthma, cystic fibrosis, stroke, acute bronchitis, chronic bronchitis, acute bronchiolitis, chronic bronchiolitis, osteoarthritis, gout, spondyloarthropathy, ankylosing spondylitis, Reiter's syndrome, psoriatic arthropathy, enteropathic spondylitis, juvenile arthropathy, juvenile ankylosing spondylitis, reactive arthropathy, infectious arthritis, post-infectious arthritis, gonococcal arthritis, tuberculous arthritis, viral arthritis, fungal arthritis, syphilitic arthritis, Lyme disease, arthritis associated with vasculitic syndrome, polyarteritis nodosa, hypersensitivity vasculitis, Lou Gehrig's granulomatosis, rheumatic polymyalgia, articular cell arteritis, calcium crystal deposition arthropathy, pseudogout, non-articular rheumatism, bursitis, tenosynovitis, epicondylitis, neuropathic joint disease (charcot and joint), hemorrhagic arthrosis (hemarthrosis), Henoch-Schönlein purpura, hypertrophic osteoarthropathy, multicentric reticulohistiocytoma, sarcoidosis, hemochromatosis, sickle cell anemia, hemoglobinopathy, hyperlipoproteinemia, hypogammaglobulinemia, familial Mediterranean fever, Behcet's disease, systemic lupus erythematosus, relapsing fever, psoriasis, multiple sclerosis, sepsis, septic shock, multiorgan dysfunction syndrome, acute respiratory distress syndrome, chronic obstructive pulmonary disease, acute lung injury, and broncho-pulmonary dysplasia.

7. The pharmaceutical composition of claim 6, wherein the inflammatory bowel disease is one or more selected from the group consisting of ulcerative colitis, Crohn's disease, Behcet's disease, collagenous colitis, lymphocytic colitis, ischemic colitis, hemorrhagic rectal ulcers, ileal pouchitis, and diversion colitis.

8. The pharmaceutical composition of claim 1, wherein the pharmaceutical composition has one or more effects selected from the group consisting of
i) reducing intestinal epithelial barrier damage;
ii) reducing intestinal tissue infiltration of immune cells;
iii) inhibiting a reduction in the number of goblet cells;
iv) inhibiting cecal weight loss;
v) inhibiting a reduction in intestinal length;
vi) inhibiting inflammatory cytokine production;
vii) promoting anti-inflammatory cytokine production;
viii) enhancing mucus secretion by goblet cells;
ix) promoting tight junction formation in intestinal epithelial cells; and
x) increasing the number of *Bifidobacterium* in the intestine.

9. A kit for preventing or treating inflammatory diseases, comprising the pharmaceutical composition of any one of claims 1 to 8.

10. A method of preventing or treating inflammatory diseases, comprising administering one or more selected from the group consisting of EVs derived from a *Roseburia* spp. strain and EVs derived from a *Bifidobacterium* spp. strain to a subject in need thereof.

11. A food composition for preventing or ameliorating inflammatory diseases, comprising one or more selected from the group consisting of EVs derived from a *Roseburia* spp. strain and EVs derived from a *Bifidobacterium* spp. strain as an active ingredient.

12. The food composition of claim 11, wherein the food composition is a health functional food composition.

13. A feed composition for preventing or ameliorating inflammatory diseases, comprising one or more selected from the group consisting of EVs derived from a *Roseburia* spp. strain and EVs derived from a *Bifidobacterium* spp. strain as an active ingredient.

14. A use of one or more selected from the group consisting of EVs derived from a *Roseburia* spp. strain and EVs derived from a *Bifidobacterium* spp. strain for preventing or treating inflammatory diseases.

15. A use of one or more selected from the group consisting of EVs derived from a *Roseburia* spp. strain and EVs derived from a *Bifidobacterium* spp. strain for preparing a therapeutic agent for inflammatory diseases.
